(19) Europäisches Patentamt
European Patent Office
Office européen des brevets

(11) EP 0 450 040 B1

(12) **FASCICULE DE BREVET EUROPEEN**

(45) Date de publication et mention de la délivrance du brevet:
**09.05.2001 Bulletin 2001/19**

(21) Numéro de dépôt: **90916095.4**

(22) Date de dépôt: **19.10.1990**

(51) Int Cl.$^7$: **A61K 7/48**, A61K 31/19, A61K 31/22, A61K 31/165

(86) Numéro de dépôt international:
**PCT/FR90/00760**

(87) Numéro de publication internationale:
**WO 91/05543 (02.05.1991 Gazette 1991/10)**

# (54) COMPOSITIONS PHARMACEUTIQUES ET COSMETIQUES DEPIGMENTANTES A BASE D'ACIDE CAFEIQUE

DEPIGMENTIERENDES PHARMAZEUTISCHES ODER KOSMETISCHES MITTEL MIT KAFFEINSÄURE

PHARMACEUTICAL AND COSMETIC DEPIGMENTATION COMPOSITIONS WITH A CAFFEIC ACID BASE

(84) Etats contractants désignés:
**AT DE FR NL**

(30) Priorité: **20.10.1989 FR 8913774**

(43) Date de publication de la demande:
**09.10.1991 Bulletin 1991/41**

(73) Titulaire: **L'OREAL**
**75008 Paris (FR)**

(72) Inventeurs:
- **CHARPIN, Isabelle**
  **F-94100 S.-Maur-des-Fossés (FR)**
- **CANDAU, Didier**
  **F-77000 Melun (FR)**
- **N'GUYEN QUANG, Lan**
  **F-92160 Antony (FR)**
- **MONTASTIER, Christiane**
  **F-78600 Maisons-Laffitte (FR)**
- **MILLECAMPS, François**
  **F-75014 Paris (FR)**

(74) Mandataire: **Stalla-Bourdillon, Bernard et al**
**NONY & ASSOCIES**
**3, rue de Penthièvre**
**75008 Paris (FR)**

(56) Documents cités:
**FR-A- 1 533 371** **FR-A- 2 390 160**
**FR-A- 2 613 622**

- **Patent Abstracts of Japan, volume 11, no. 349 (C-456)[2796] 14 novembre 1987, & JP, A, 62120312 (POLA CHEM. IND. INC.) 1er juin 1987, voir le résumé**
- **Patent Abstracts of Japan, volume 6, no. 44, (C-95)[922] 19 mars 1982, & JP, A, 56161315 (ICHIMARU BOUEKI K.K.) 11 décembre 1981, voir le résumé**
- **Patent Abstracts of Japan, volume 15, no. 1, 7 janvier 1991, & JP, A, 2255607 (PIAS ARISE KK) 16 octobre 1990, voir le résumé**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 450 040 B1

Printed by Jouve, 75001 PARIS (FR)

## Description

La présente invention a pour objet l'utilisation de l'acide caféique ou l'un de ses alkylesters ou alkylamides dans le traitement des hyperpigmentations régionales ou accidentelles ou comme actif dépigmentant dans une composition cosmétique.

On rappellera que le mécanisme de formation de la pigmentation de la peau, c'est-à-dire de la formation des mélanines est particulièrement complexe et fait intervenir schématiquement les principales étapes suivantes :

TYROSINE → DOPA → DOPAQUINONE → DOPACHROME → MELANINES

l'enzyme intervenant dans cette suite de réactions étant essentiellement la tyrosinase.

Les dépigmentants actuellement utilisés en cosmétologie sont plus particulièrement des dérivés phénoliques et en particulier l'hydroquinone ou un éther d'hydroquinone tel que le monométhyléther d'hydroquinone.

Ces composés, s'ils ont une efficacité certaine, ne sont malheureusement pas exempts d'effets secondaires, ce qui peut rendre leur emploi délicat voire dangereux.

Ainsi l'hydroquinone, dont l'emploi est d'ailleurs limité à une concentration de 2 %, est un composé particulièrement irritant et cytotoxique pour le mélanocyte, dont le remplacement, total ou partiel, a été envisagé par de nombreux auteurs.

Il a été ainsi proposé dans le brevet français n° 87.08236 (2.616.325) une composition contenant en association de l'hydroquinone et de l'acide kojique ou ses dérivés, en particulier ses sels ou esters.

Dans la demande internationale W0 85/0401, il a été également proposé, dans le même but, une composition dépigmentante contenant en association de l'hydroquinone ou son monoéther benzylique et de l'acide salicylique.

Par ailleurs on doit également citer, parmi les compositions ayant une activité dépigmentante importante, le trio de Kligman, généralement retenu comme composition de référence, qui est basé sur une combinaison de vitamine A acide, d'hydroquinone et d'un stéroïde constitué par la déxaméthasone ou l'hydrocortisone. L'utilisation de cette composition a toutefois été limitée dans la mesure où celle-ci présentait un caractère irritant élevé provoqué par la vitamine A acide, et des effets indésirables dus à l'hypervitaminose A.

Il est maintenant bien établi qu'une substance exerce une action dépigmentante si elle agit directement sur la vitalité des mélanocytes épidermiques où se déroule normalement la mélanogénèse, et/ou si elle interfère avec une des étapes de la biosynthèse des mélanines soit en inhibant un des enzymes impliqués, soit en s'intercalant comme analogue structural dans la voie de synthèse qui peut ainsi être bloquée, d'où l'effet dépigmentant.

L'utilisation de substances dépigmentantes topiques présentant une bonne efficacité et étant inoffensive, est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou secondaires à la contraception oestro-progestative, les hyperpigmentations localisées par hyperactivité et prolifération mélanocytaire bénigne telles que les taches pigmentaires séniles dites lentigos actiniques, les hyperpigmentations accidentelles telles que la photosensibilisation et la cicatrisation post-lésionnelle, ainsi que certaines leucodermies telles que le vitiligo où, à défaut de pouvoir repigmenter la peau lésée, on achève de dépigmenter les zones de peau normale résiduelle pour donner à l'ensemble de la peau une teinte blanche homogène.

Après diverses études effectuées sur de nombreuses substances soit seules soit en association, on a constaté de façon tout à fait surprenante et inattendue que l'acide caféique ou ses dérivés, notamment ses esters et amides, avaient une action dépigmentante particulièrement intéressante, leur efficacité étant essentiellement due à un effet d'inhibition de l'activité tyrosinasique, aboutissant à la formation limitée de dopachrome et donc des mélanines.

Cette nouvelle propriété de l'acide caféique et de ses dérivés n'avait jusqu'à présent jamais été mise en évidence malgré les nombreuses études qui ont été réalisées sur cet acide, et ses esters.

On peut à cet égard citer le brevet français 7.814.296 (2.390.160) qui décrit des esters de l'acide caféique notamment son ester éthylique comme agents anti-inflammatoires dans des produits solaires.

L'état de la technique est également représenté par la demande de brevet japonais 56-161.315 qui décrit l'utilisation d'esters stéroïdiques de l'acide férulique, ce dernier étant le dérivé 3-méthoxy de l'acide caféique, comme étant des inhibiteurs de l'activité tyrosinase utiles dans le traitement des dermatites.

On peut citer en outre, l'utilisation décrite dans la demande de brevet japonais 62-120312, de l'acide isoférulique ou de ses dérivés et en particulier de ses alkylesters comme inhibiteurs de la tyrosinase, dans des compositions d'embellissement notamment destinées à atténuer les taches et les taches de rousseur.

La présente invention a donc pour objet, selon un premier mode de réalisation, l'utilisation de l'acide caféique ou de l'un de ses alkylesters ou alkylamides pour la préparation d'une composition pharmaceutique destinée au traitement par voie topique des hyperpigmentations régionales ou accidentelles.

Selon un deuxième mode de réalisation, l'invention a pour objet l'utilisation de l'acide caféique ou l'un de ses

alkylesters ou alkylamides comme actif dépigmentant dans une composition cosmétique par voie topique.

Parmi les alkylesters de l'acide caféique on peut notamment mentionner les composés de formule :

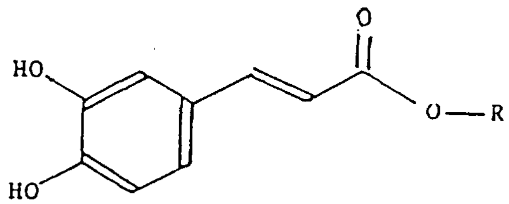

(1)

R représentant un radical alkyle ayant de 1 à 8 atomes de carbone, et de préférence le radical méthyle.

Parmi les alkylamides de l'acide caféique on peut notamment mentionner les composés de formule :

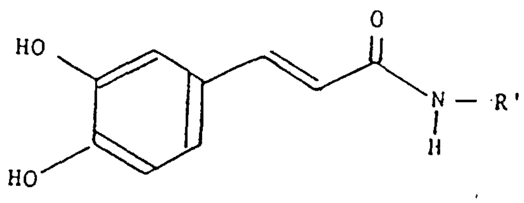

(2)

R' représentant un radical alkyle ayant de 1 à 8 atomes de carbone, et de préférence un alkyle ayant de 6 à 8 atomes de carbone ; l'alkylamide ayant 6 atomes de carbone est plus particulièrement préféré.

Dans les compositions selon l'invention, la concentration en acide caféique ou en l'un de ses alkylesters ou alkylamides, est généralement comprise entre 0,1 et 8 % et de préférence entre 1 et 3 % en poids.

Le véhicule des compositions selon l'invention peut être notamment une solution aqueuse ou hydroalcoolique, une émulsion du type huile-dans-l'eau ou eau-dans-l'huile, un gel émulsionné ou encore un système biphasé.

De préférence les compositions selon l'invention se présentent sous forme d'une lotion, d'une crème, d'un lait, d'un gel, d'un masque, de microsphères ou nanosphères ou de dispersions vésiculaires. Dans le cas de ces dernières au moins un des actifs peut être liposomé.

On a par ailleurs constaté que, de façon tout à fait surprenante, lorsque l'acide caféique ou l'un de ses alkylesters ou alkylamides était associé à une substance choisie parmi l'hydroquinone ou l'un de ses éthers, l'acide kojique (hydroxy-5 hydroxyméthyl-2 H-4 pyranone-4), un sel hydrosoluble de cuivre ou un mélange de ces substances, on observait une efficacité accrue de l'action dépigmentante, cette activité ne pouvant être attribuée qu' à un effet de synergie.

Lorsque les compositions selon l'invention contiennent de l'hydroquinone ou un éther d'hydroquinone tel que le monométhyléther d'hydroquinone, la concentration est généralement comprise entre 0,2 et 6 % mais de préférence entre 0,2 et 2 % en poids, ceci afin d'éviter ses effets secondaires.

Lorsque les compositions contiennent de l'acide kojique, la concentration est généralement comprise entre 0,1 et 3 % en poids et de préférence entre 0,5 et 2 %.

Lorsque les compositions contiennent un sel hydrosoluble de cuivre, celui-ci se trouve à une concentration molaire en Cu II équivalente à celle de l'acide caféique ou de ses dérivés.

Il s'agit en particulier du gluconate ou du sulfate de cuivre, mais de préférence du gluconate de cuivre à une concentration comprise entre 0,2 et 20 % en poids et de préférence entre 2,5 et 7,5 %.

Selon un mode de réalisation préféré, on introduit en outre dans la composition, un agent kératolytique à une concentration comprise par exemple entre 0,5 et 10 % en poids, et de préférence entre 1 et 3 %.

Cet agent kératolytique peut être notamment l'urée ou un hydroxyacide carboxylique tel que l'acide glycolique ou préférentiellement l'acide salicylique ou ses dérivés et en particulier l'acide n-octanoyl-5 salicylique.

Selon l'invention les compositions peuvent donc être du type binaire, ternaire ou quaternaire.

Parmi les associations faisant partie de l'invention, on peut citer notamment :

- acide caféique + acide kojique + acide salicylique ou l'un de ses dérivés
- acide caféique + acide kojique + hydroquinone
- acide caféique + acide kojique + gluconate de cuivre
- acide caféique + acide kojique + hydroquinone + gluconate de cuivre
- acide caféique + hydroquinone
- acide caféique + hydroquinone + gluconate de cuivre

- acide caféique + gluconate de cuivre
- acide caféique + acide kojique,

les quatre premières étant particulièrement préférées.

L'invention a également pour objet un procédé de préparation des compositions décrites ci-dessus, par mélange, selon les méthodes usuelles, du ou des ingrédients actifs avec le véhicule et les autres ingrédients éventuellement présents.

L'invention a en outre pour objet l'utilisation de l'acide caféique (ou un des ses alkylesters ou alkylamides), éventuellement en association avec les autres ingrédients mentionnés ci-dessus, comme principe actif dans la préparation d'une composition cosmétique ou pharmaceutique à action dépigmentante.

Les compositions selon l'invention peuvent bien entendu contenir d'autres ingrédients usuels en cosmétologie ou en dermo-pharmacie, tels que des agents humectants, des conservateurs, des colorants, des parfums, des agents de pénétration tels que le monoéthyléther de diéthylèneglycol, etc...

Ces compositions sont appliquées par voie topique chez l'homme, en quantité correspondant aux doses d'application usuelles pour le type de composition considéré (gel, crème, lotion, etc...). Par exemple, dans le cas d'une crème, on utilise de 0,5 à 3 mg, et notamment de 1 à 2 mg de crème par $cm^2$ de peau et par application, à raison d'une ou deux applications par jour.

ETUDE "IN VITRO"

On a étudié "in vitro" l'efficacité de diverses associations selon l'invention en dosant par spectrophotométrie visible (475 nm) la quantité de dopachrome formée au cours de la chaîne de réactions d'oxydation de la tyrosine. Ces réactions d'oxydation sont catalysées "in vitro" par la tyrosinase de champignons, en présence d'un co-substrat (par exemple la L-dopa en petite quantité) pour initier la réaction d'hydroxylation de la L-tyrosine en L-dopa, laquelle est ensuite oxydée catalytiquement en dopaquinone, puis en dopachrome, produit intermédiaire avant les réactions d'oxydation non enzymatiques aboutissant à la formation des mélanines.

On suit donc la concentration en dopachrome formée au cours du temps, en présence de divers inhibiteurs de l'activité tyrosinasique.

Les concentrations en inhibiteurs sont exprimées en % molaire par rapport à la concentration en tyrosine présente dans le milieu réactionnel.

L'effet d'inhibition peut s'exprimer, par rapport à la courbe témoin (absence d'inhibiteur), par un abaissement de la quantité de dopachrome formée et/ou par un ralentissement de la cinétique de formation de la dopachrome.

Dans le premier cas, cet abaissement se traduit par une diminution du niveau maximum de densité optique atteint (cas des produits actifs qui sont plutôt limiteurs de dopachrome formée), et dans l'autre cas, par une diminution de la pente tangente à l'origine (cas des produits actifs qui sont plutôt ralentisseurs de formation de la dopachrome).

Les courbes des figures I à V illustrent l'effet inhibiteur de différentes substances et associations de substances selon l'invention.

Protocole expérimental

Réactifs :

A - Tampon phosphate 0,1M pH = 6,5
B - Solution mère de L-tyrosine à $2.10^{-3}$M dans A
C - Solution mère de L-dopa à $10^{-4}$M dans A
D - Solution mère de tyrosinase de champignons à 2.400 unités/ml dans A
E - Solution mère de l'inhibiteur à $10^{-2}$M dans A

(Les solutions C et D sont à préparer le jour même)

Mesures

- cuve de référence : 3ml de A
- cuve d'essai : 1ml de B

0,1ml de C
1,85ml de A+E

- homogénéiser et équilibrer à 25°C
- ajouter 0,05ml de D
- mélanger rapidement et déclencher la cinétique d'absorbance à 475nm.

On va maintenant donner à titre d'illustration n'ayant aucun caractère limitatif plusieurs exemples de compositions selon l'invention :

EXEMPLE 1 : Crème dépigmentante

| | |
|---|---|
| Acide caféique | 1,27 % |
| Acide kojique | 1 % |
| Acide n-octanoyl-5-salicylique | 2 % |
| Triéthanolamine | 1,3 % |
| Huile de jojoba | 3 % |
| Mélange d'éthyl-2 hexanoate de cétostéaryle et de myristate d'isopropyle | 3 % |
| Isoparaffine hydrogénée | 3 % |
| Acide stéarique | 2 % |
| Alcool cétylstéarylique | 1,5 % |
| Myristate d'isopropyle | 3 % |
| Conservateur | 0,4 % |
| Tampon | 0,71 % |
| Sel disodique de l'acide éthylène diamine-tétracétique hydraté (2 $H_2O$) | 0,1 % |
| Filtres UVA-UVB | 2 % |
| Parfum | 0,4 % |
| Polymère carboxyvinylique | 0,6 % |
| Monoéthyl éther de diéthylèneglycol | 7,5 % |
| Eau déminéralisée stérile | 58,22 % |
| Stéarate de polyéthylèneglycol | 3 % |
| Mélange de monostéarate de glycéryle et de stéarate de polyéthylèneglycol | 3 % |
| Mélange d'alcool cétylstéarylique et d'alcool cétylstéarylique oxyéthyléné | 3 % |

Cette crème a été testée cliniquement sur un groupe de 21 sujets âgés de 45 à 65 ans, présentant des taches de sénescence au niveau des mains.
Le traitement, d'une durée de 2 mois, consistait en une application biquotidienne sur une main, l'autre main servant de témoin. On utilisait 2 mg de crème par $cm^2$ de peau à chaque application.
A la fin du traitement, une évaluation quantitative a été réalisée à l'aide d'un chromamètre CR 200 (MINOLTA) au niveau des taches traitées et des taches non traitées.
On calcule la variation absolue de couleur, toutes nuances confondues, entre les temps T = 0 et T = 2 mois, de la manière suivante :

$$\Delta E = \sqrt{\Delta L^2 + \Delta a^2 + \Delta b^2},$$

L, a et b étant les coordonnées définissant l'espace colorimétrique utilisé (Δ signifie:variation de).
Les résultats sont les suivants :

| | Main traitée | Main non traitée |
|---|---|---|
| Δ E | 2,31 | 2,8 |
| % d'inhibition par rapport à la main non traitée | 21,6 | |

L'efficacité subjective de cette crème est de 60 %.

La tolérance est de 100 %.

EXEMPLE 2 : Crème dépigmentante

| | |
|---|---|
| Acide caféique | 1,27 % |
| Acide kojique | 1 % |
| Hydroquinone | 2 % |
| Triéthanolamine | 1,3 % |
| Huile de jojoba | 3 % |
| Mélange d'éthyl-2 hexanoate de cétostéaryle et de myristate d'isopropyle | 3 % |
| Isoparaffine hydrogénée | 3 % |
| Acide stéarique | 2 % |
| Alcool cétylstéarylique | 1,5 % |
| Myristate d'isopropyle | 3 % |
| Conservateur | 0,4 % |
| Tampon | 0,71 % |
| Sel disodique de l'acide éthylène diamine-tétracétique hydraté ($2H_2O$) | 0,1 % |
| Filtres UVA-UVB | 2 % |
| Parfum | 0,4 % |
| Polymère carboxyvinylique | 0,6 % |
| Monoéthyl éther de diéthylèneglycol | 7,5 % |
| Eau déminéralisée stérile | 58,22 % |
| Stéarate de polyéthylèneglycol | 3 % |
| Mélange de mono-stéarate de glycéryle et de stéarate de polyéthylèneglycol | 3 % |
| Mélange d'alcool cétylstéarylique et d'alcool cétylstéarylique oxyéthyléné | 3 % |

EXEMPLE 3 : Crème dépigmentante

| | |
|---|---|
| Acide caféique | 1,27 % |
| Hydroquinone | 2 % |
| Triéthanolamine | 1,3 % |
| Huile de jojoba | 3 % |
| Mélange d'éthyl-2 hexanoate de cétostéaryle et de myristate d'isopropyle | 3 % |
| Isoparaffine hydrogénée | 3 % |
| Acide stéarique | 2 % |
| Myristate d'isopropyle | 3 % |
| Alcool cétylstéarylique | 1,5 % |
| Conservateur | 0,4 % |
| Tampon | 0,71 % |
| Sel disodique de l'acide éthylène diamine-tétracétique hydraté ($2H_2O$) | 0,1 % |
| Filtres UVA-UVB | 2 % |
| Parfum | 0,4 % |
| Polymère carboxyvinylique | 0,6 % |
| Monoéthyl éther de diéthylèneglycol | 7,5 % |
| Eau déminéralisée stérile | 61,22 % |
| Stéarate de polyéthylèneglycol | 3 % |
| Mélange de mono-stéarate de glycéryle et de stéarate de polyéthylèneglycol | 3 % |

(suite)

| | |
|---|---|
| Mélange d'alcool cétylstéarylique et d'alcool cétylstéarylique oxyéthyléné | 1 % |

EXEMPLE 4 : Lotion dépigmentante

| | |
|---|---|
| Acide caféique | 2,35 % |
| Hydroquinone | 2 % |
| Gluconate de cuivre | 6 % |
| Tampon | 0,3 % |
| Sel di-sodique de l'acide éthylène diamine-tétracétique hydraté (2 $H_2O$) | 4,1 % |
| Acide éthylène diamino tétraméthylène phosphonique | 0,1 % |
| Diméthylsulfoxyde | 42,8 % |
| Eau déminéralisée stérile | 42,35 % |

EXEMPLE 5 : Lotion dépigmentante

| | |
|---|---|
| Acide caféique | 1,6 % |
| Acide kojique | 1,25 % |
| Hydroquinone | 2 % |
| Gluconate de cuivre | 4 % |
| Acide éthylène diamine tétraméthylène phosphonique | 0,1 % |
| Tampon | 0,3 % |
| Sel disodique de l'acide éthylène diamine-tétracétique hydraté (2 $H_2O$) | 3,3 % |
| Diméthylsulfoxyde | 43,9 % |
| Eau déminéralisée stérile | 43,55 % |

EXEMPLE 6 : Lotion dépigmentante

| | |
|---|---|
| Acide caféique | 2,5 % |
| Acide kojique | 2 % |
| Gluconate de cuivre | 6,5 % |
| Tampon | 0,3 % |
| Sel disodique de l'acide éthylène diamine-tétracétique hydraté (2 $H_2O$) | 5,6 % |
| Acide éthylène diamine tétraméthylène phosphonique | 0,1 % |
| Diméthylsulfoxyde | 41,6 % |
| Eau déminéralisée stérilisée | 41,4 % |

EXEMPLE 7 : Lotion dépigmentante

| | |
|---|---|
| Acide caféique | 8 % |
| Tampon | 0,15 % |
| Acide éthylène diamine tétraméthylène phosphonique | 0,1 % |
| Diméthylsulfoxyde | 68,8 % |
| Eau déminéralisée stérile | 22,95 % |

EXEMPLE 8 :

On prépare selon l'invention une dispersion vésiculaire sous forme d'une crème en procédant de la façon suivante :

Dans un bécher on introduit les produits suivants :

- Lipide amphiphile non ionique de formule :

$$RO-\!\!\!-\left[C_3H_5(OH)O\right]_{\bar{n}}-\!\!\!-H$$

dans laquelle : $-C_3H_5$ (OH) O- représente une des structures suivantes ou un mélange de celles-ci :

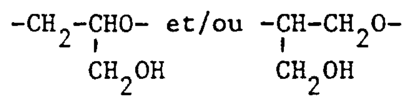

R étant un radical hexadécyle,
et $\bar{n}$ ayant une valeur statistique
moyenne égale à 3 3,75 g

- Cholestérol 3,75 g

On procède au mélange de ces deux produits par fusion à une température de 110°C sous atmosphère d'azote, puis on ramène la température du mélange fondu à 90°C.
A celui-ci on ajoute alors :

- Acide n-octanoyl-5-salicylique 0,5 g

Après homogénéisation du mélange on ajoute alors les produits suivants :

- Glycérine 3,0 g
- Eau déminéralisée 19,3 g

On homogénéise ensuite le mélange à une température de 70°C à l'aide d'un ultradisperseur du type VIRTIS.
On ajoute alors :

- Acide kojique 1,0 g
- Eau déminéralisée 23,02 g

Après homogénéisation du mélange à l'aide de l'ultradisperseur, à une température de 40°C, on ajoute ensuite :

- Acide caféique 1,27 g
- Monoéthyléther de diéthylèneglycol 7,5 g puis homogénéise à nouveau à la même température.

On obtient ainsi une dispersion de vésicules lipidiques dont la taille moyenne est d'environ 0,2 micron.
Après avoir amené la température à 25°C, on ajoute alors :

- Huile de vaseline 14 g
- Huile de silicone volatile 10 g

On homogénéise à l'aide de l'ultradisperseur et on ajoute enfin les produits suivants :

- Parfum 0,4 g
- Polymère carboxyvinylique vendu sous la dénomination de "CARBOPOL 940" par la Société GOODRICH 0,4 g

- Triéthanolamine 1,5 g
- Parahydroxybenzoate de méthyle 0,2 g
- Eau déminéralisée 10,41 g

On obtient ainsi une crème de couleur beige.

EXEMPLE 9 :

On prépare selon l'invention une dispersion vésiculaire sous forme d'une crème en procédant de la façon suivante :

- Lipide amphiphile non ionique de formule :

$$RO-\left[C_3H_5(OH)\ O\right]_{\bar{n}}-H$$

dans laquelle $C_3H_5(OH)O$- représente une des structures suivantes ou un mélange de celles-ci :

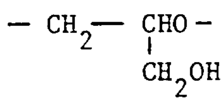
et/ou
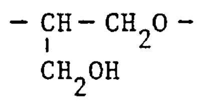

R étant un radical hexadécyle,
et $\bar{n}$ ayant une valeur statistique moyenne
égale à 3 3,75 g
- Cholestérol 3,75 g

On procède au mélange de ces deux produits par fusion à une température de 110°C sous atmosphère d'azote, puis on ramène la température du mélange fondu à 90°C.
A celui-ci on ajoute alors :

- Acide n-octanoyl salicylique 0, 5 g

Après homogénéisation du mélange on ajoute alors les produits suivants :

- Glycérine 3, 0 g
- Eau déminéralisée 14, 5 g

On homogénéise ensuite le mélange à une température de 70°C à l'aide d'un ultradisperseur du type VIRTIS.
On ajoute alors :

- Hydroquinone 2 g
- Métabisulfite de sodium 0,17 g
- Sulfite de sodium 0,27 g
- Acide citrique 0, 2 g
- Eau déminéralisée 26,12 g

Après homogénéisation du mélange à l'aide de l'ultradisperseur, à une température de 40°C, on ajoute ensuite :

- Acide caféique 1,27 g
- Monoéthyléther de diéthylèneglycol 7,5 g

puis homogénéise à nouveau à la même température.
On obtient ainsi une dispersion de vésicules lipidiques dont la taille moyenne est d'environ 0,2 micron.

Après avoir amené la température à 25°C, on ajoute alors :

- Huile de vaseline 14 g
- Huile de silicone volatile 10 g

On homogénéise à l'aide de l'ultradisperseur et on ajoute enfin les produits suivants :

- Parfum 0,4 g
- Polymère carboxyvinylique vendu sous la dénomination de "CARBOPOL 940" par la Société GOODRICH 0,4 g
- Triéthanolamine 1,4 g
- Parahydroxybenzoate de méthyle 0,2 g
- Eau déminéralisée 10,51 g

On obtient ainsi une crème de couleur beige.

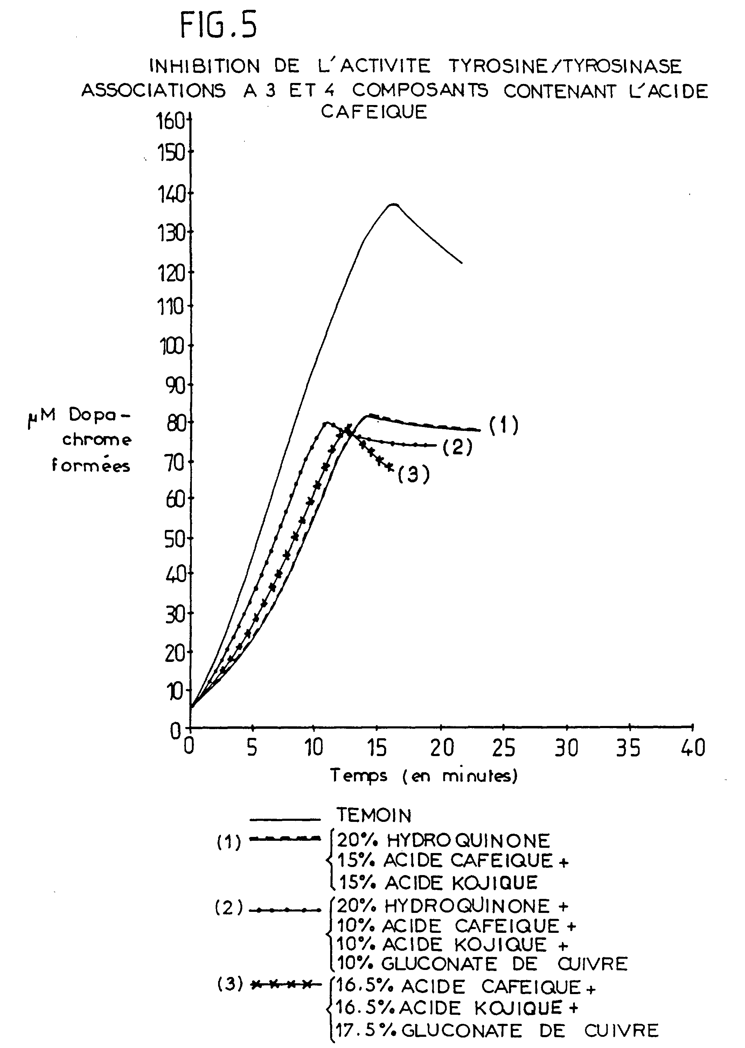

## Revendications

1. Utilisation de l'acide caféique ou l'un de ses alkylesters ou alkylamides pour la préparation d'une composition pharmaceutique destinée au traitement par voie topique des hyperpigmentations régionales ou accidentelles.

2. Utilisation de l'acide caféique ou l'un de ses alkylesters ou alkylamides comme actif dépigmentant dans une composition cosmétique par voie topique.

3. Utilisation selon la revendication 1 ou 2, caractérisée par le fait que la composition contient de 0,1 à 8 % en poids d'acide caféique ou de l'un de ses alkylesters ou alkylamides par rapport au poids total de la composition.

4. Utilisation selon l'une quelconque des revendications précédentes, caractérisée par le fait que les alkylesters de l'acide caféique répondent à la formule générale suivante :

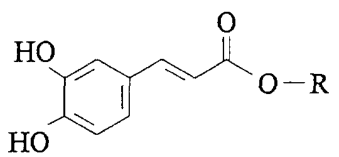

(I)

dans laquelle :
R représente un radical alkyle ayant de 1 à 8 atomes de carbone.

5. Utilisation selon l'une quelconque des revendications 1 à 3, caractérisée par le fait que les alkylamides de l'acide caféique répondent à la formule générale suivante :

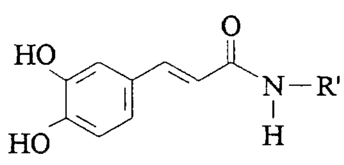

(II)

dans laquelle :
o R' représente un radical alkyle ayant de 1 à 8 atomes de carbone.

6. Utilisation selon l'une quelconque des revendications précédentes, caractérisée par le fait que la composition contient en outre une substance choisie parmi l'hydroquinone, ou un éther d'hydroquinone, l'acide kojique, un sel hydrosoluble de cuivre ou un mélange de ces substances.

**7.** Utilisation selon la revendication 6, caractérisée par le fait que la composition contient de l'hydroquinone ou un éther d'hydroquinone à une concentration comprise entre 0,2 et 6 % en poids par rapport au poids total de la composition.

**8.** Utilisation selon la revendication 6, caractérisée par le fait que le sel hydrosoluble de cuivre est le gluconate de cuivre.

**9.** Utilisation selon l'une quelconque des revendications précédentes, caractérisée par le fait que la composition contient en outre au moins un agent kératolytique.

**10.** Utilisation selon la revendication 9, caractérisée par le fait que ledit agent kératolytique est choisi parmi l'urée et un acide carboxylique hydroxylé.

**11.** Utilisation selon la revendication 10, caractérisée par le fait que ledit acide hydroxylé est choisi parmi l'acide salicylique ou l'un de ses dérivés ou l'acide glycolique.

**12.** Utilisation selon la revendication 11, caractérisée par le fait que le dérivé d'acide salicylique est l'acide n-octanoyl-5 salicylique.

**13.** Utilisation selon l'une quelconque des revendications 9 à 12, caractérisée par le fait que ledit agent kératolytique est présent à une concentration comprise entre 0,5 et 10 % en-poids par rapport au poids total de la composition.

**14.** Utilisation selon l'une quelconque des revendications précédentes, caractérisée par le fait que la composition contient une association d'acide caféique, d'acide kojique et d'acide n-octanoyl-5-salicylique.

**15.** Composition pharmaceutique ou cosmétique à action dépigmentante par voie topique, caractérisée par le fait qu'elle contient, dans un véhicule approprié, une quantité efficace d'un alkylamide de l'acide caféique répondant à la formule générale suivante :

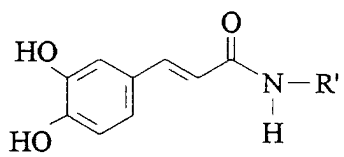

(II)

dans laquelle :
R' représente un radical alkyle ayant de 1 à 8 atomes de carbone.

**16.** Composition selon la revendication 15, caractérisée par le fait qu'elle contient de 0,1 à 8 % en poids d'alkylamide de l'acide caféique par rapport au poids total de la composition.

**17.** Composition selon la revendication 15 ou 16, caractérisée par le fait qu'elle contient en outre une substance choisie parmi l'hydroquinone ou un éther d'hydroquinone, l'acide kojique, un sel hydrosoluble de cuivre, ou un mélange de ces substances.

**18.** Composition selon l'une quelconque des revendications 15 à 17, caractérisée par le fait qu'elle contient de l'hydroquinone ou un éther d'hydroquinone à une concentration comprise entre 0,2 et 6 % en poids par rapport au poids total de la composition.

**19.** Composition selon l'une quelconque des revendications 15 à 17, caractérisée par le fait qu'elle contient de l'acide kojique à une concentration comprise entre 0,1 et 3 % en poids par rapport au poids total de la composition.

**20.** Composition selon l'une quelconque des revendications 15 à 17, caractérisée par le fait qu'elle contient ledit sel hydrosoluble de cuivre à une concentration molaire équivalente à celle de l'alkylamide de l'acide caféique.

**21.** Composition selon l'une quelconque des revendications 15 à 20, caractérisée par le fait qu'elle contient en outre

au moins un agent kératolytique.

**22.** Composition selon la revendication 21, caractérisée par le fait que ledit agent kératolytique est choisi parmi l'urée et un acide carboxylique hydroxylé.

**23.** Composition selon la revendication 21 ou 22, caractérisée par le fait que ledit agent kératolytique est présent à une concentration comprise entre 0,5 et 10 % en poids par rapport au poids total de la composition.

**24.** Composition pharmaceutique ou cosmétique ayant une action dépigmentante par voie topique, caractérisée par le fait qu'elle contient, dans un véhicule approprié, une quantité efficace d'acide caféique ou de l'un de ses alkylesters ou alkylamides en association avec une substance choisie parmi l'hydroquinone ou un éther d'hydroquinone, l'acide kojique, un sel hydrosoluble de cuivre, ou un mélange de ces substances.

**25.** Conlposition selon la revendication 24, caractérisée par le fait qu'elle contient de 0,1 à 8 % en poids d'acide caféique ou de l'un de ses alkylesters ou alkylamides.

**26.** Composition selon la revendication 24 ou 25 caractérisée par le fait qu'elle contient de 0,2 à 8 % en poids d'une substance choisie parmi l'hydroquinone ou un éther d'hydroquinone, l'acide kojique, un sel hydrosoluble de cuivre ou un mélange de ces substances.

**27.** Procédé de traitement cosmétique en vue d'améliorer l'aspect esthétique de la peau, caractérisé par le fait qu'il consiste à appliquer sur la peau une quantité efficace d'une composition définie selon l'une quelconque des revendications 15 à 26.

**Claims**

**1.** Use of caffeic acid or an alkyl ester or alkylamide thereof for the preparation of a pharmaceutical composition intended for the topical treatment of regional or accidental hyperpigmentations.

**2.** Use of caffeic acid or an alkyl ester or alkylamide thereof as a depigmenting active agent in a topical-route cosmetic composition.

**3.** Use according to Claim 1 or 2, characterized in that the composition contains from 0.1% to 8% by weight of caffeic acid or an alkyl ester or alkylamide thereof relative to the total weight of the composition.

**4.** Use according to any one of the preceding claims, characterized in that the alkyl esters of caffeic acid correspond to the following general formula:

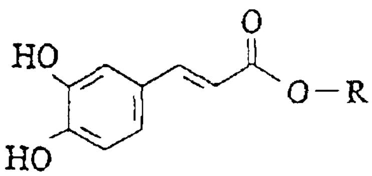

(I)

in which:
R represents an alkyl radical containing from 1 to 8 carbon atoms.

**5.** Use according to any one of Claims 1 to 3, characterized in that the alkylamides of caffeic acid correspond to the following general formula:

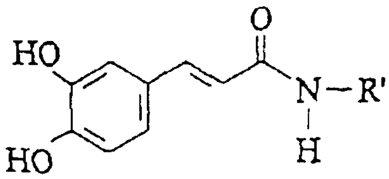

(II)

in which:
R' represents an alkyl radical containing from 1 to 8 carbon atoms.

**6.** Use according to any one of the preceding claims, characterized in that the composition also contains a substance chosen from hydroquinone, a hydroquinone ether, kojic acid and a water-soluble copper salt, or a mixture of these substances.

**7.** Use according to Claim 6, characterized in that the composition contains hydroquinone or a hydroquinone ether at a concentration of between 0.2% and 6% by weight relative to the total weight of the composition.

**8.** Use according to Claim 6, characterized in that the water-soluble copper salt is copper gluconate.

**9.** Use according to any one of the preceding claims, characterized in that the composition also contains at least one keratolytic agent.

**10.** Use according to Claim 9, characterized in that the said keratolytic agent is chosen from urea and a hydroxycarboxylic acid.

**11.** Use according to Claim 10, characterized in that the said hydroxy acid is chosen from salicylic acid or a derivative thereof, or glycolic acid.

**12.** Use according to Claim 11, characterized in that the salicylic acid derivative is 5-n-octanoylsalicylic acid.

**13.** Use according to any one of Claims 9 to 12, characterized in that the said keratolytic agent is present at a concentration of between 0.5% and 10% by weight relative to the total weight of the composition.

**14.** Use according to any one of the preceding claims, characterized in that the composition contains a combination of caffeic acid, kojic acid and 5-n-octanoylsalicylic acid.

**15.** Pharmaceutical or cosmetic composition with a topical-route depigmenting action, characterized in that it contains, in a suitable vehicle, an effective amount of an alkylamide of caffeic acid corresponding to the following general formula:

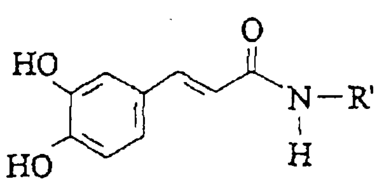

(II)

in which:
R' represents an alkyl radical containing from 1 to 8 carbon atoms.

**16.** Composition according to Claim 15, characterized in that it contains from 0.1% to 8% by weight of caffeic acid alkylamide relative to the total weight of the composition.

**17.** Composition according to Claim 15 or 16, characterized in that it also contains a substance chosen from hydroquinone, a hydroquinone ether, kojic acid and a water-soluble copper salt, or a mixture of these substances.

**18.** Composition according to any one of Claims 15 to 17, characterized in that it contains hydroquinone or a hydroquinone ether at a concentration of between 0.2% and 6% by weight relative to the total weight of the composition.

**19.** Composition according to any one of Claims 15 to 17, characterized in that it contains kojic acid at a concentration of between 0.1% and 3% by weight relative to the total weight of the composition.

**20.** Composition according to any one of Claims 15 to 17, characterized in that it contains the said water-soluble copper salt at a molar concentration equivalent to that of the caffeic acid alkylamide.

**21.** Composition according to any one of Claims 15 to 20, characterized in that it also contains at least one keratolytic agent.

**22.** Composition according to Claim 21, characterized in that the said keratolytic agent is chosen from urea and a hydroxycarboxylic acid.

**23.** Composition according to Claim 21 or 22, characterized in that the said keratolytic agent is present at a concentration of between 0.5% and 10% by weight relative to the total weight of the composition.

**24.** Pharmaceutical or cosmetic composition with a topical-route depigmenting action, characterized in that it contains, in a suitable vehicle, an effective amount of caffeic acid or an alkyl ester or alkylamide thereof in combination with a substance chosen from hydroquinone, a hydroquinone ether, kojic acid and a water-soluble copper salt, or a mixture of these substances.

**25.** Composition according to Claim 24, characterized in that it contains from 0.1% to 8% by weight of caffeic acid or an alkyl ester or alkylamide thereof.

**26.** Composition according to Claim 24 or 25, characterized in that it contains from 0.2% to 8% by weight of a substance chosen from hydroquinone, a hydroquinone ether, kojic acid and a water-soluble copper salt, or a mixture of these substances.

**27.** Cosmetic treatment process for improving the aesthetic appearance of the skin, characterized in that it consists in applying an effective amount of a composition defined according to any one of Claims 15 to 26 to the skin.

### Patentansprüche

**1.** Verwendung von Kaffeesäure oder einem ihrer Alkylester oder Alkylamide zur Herstellung einer pharmazeutischen Zubereitung zur Behandlung auf topischem Wege von lokalisierten oder auf Beschädigungen beruhenden Hyperpigmentierungen.

**2.** Verwendung von Kaffeesäure oder einem ihrer Alkylester oder Alkylamide als depigmentierender Wirkstoff in einer kosmetischen Zubereitung für die topische Anwendung.

**3.** Verwendung gemäss Anspruch 1 oder 2, dadurch gekennzeichnet, daß die Zubereitung 0,1 bis 8 Gew.-% Kaffeesäure oder eines ihrer Alkylester oder Alkylamide enthält, bezogen auf das Gesamtgewicht der Zubereitung.

**4.** Verwendung gemäss einem der vorstehenden Ansprüche, dadurch gekennzeichnet, daß die Alkylester der Kaffeesäure der folgenden allgemeinen Formel entsprechen:

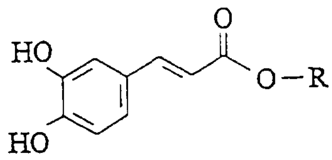

(I)

worin:
R einen Alkylrest mit 1 bis 8 Kohlenstoffatomen darstellt.

**5.** Verwendung gemäss einem der Ansprüche 1 bis 3, dadurch gekennzeichnet, daß die Alkylamide der Kaffeesäure der folgenden allgemeinen Formel entsprechen:

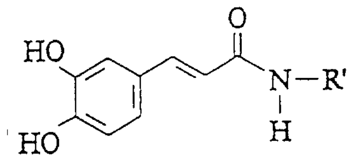
(II)

worin:
R' einen Alkylrest mit 1 bis 8 Kohlenstoffatomen darstellt.

**6.** Verwendung gemäss einem der vorstehenden Ansprüche, dadurch gekennzeichnet, daß die Zubereitung zusätzlich eine Substanz enthält, ausgewählt unter Hydrochinon, einem Hydrochinonether, Kojisäure, einem wasserlöslichen Kupfersalz oder einer Mischung dieser Substanzen.

**7.** Verwendung gemäss Anspruch 6, dadurch gekennzeichnet, daß die Zubereitung Hydrochinon oder einen Hydrochinonether in einer Konzentration zwischen 0,2 und 6 Gew.-% enthält, bezogen auf das Gesamtgewicht der Zubereitung.

**8.** Verwendung gemäss Anspruch 6, dadurch gekennzeichnet, daß es sich bei dem wasserlöslichen Kupfersalz um das Kupfergluconat handelt.

**9.** Verwendung gemäss einem der vorstehenden Ansprüche, dadurch gekennzeichnet, daß die Zubereitung zusätzlich mindestens ein keratolytisches Mittel enthält.

**10.** Verwendung gemäss Anspruch 9, dadurch gekennzeichnet, daß das keratolytische Mittel ausgewählt ist unter Harnstoff und einer Hydroxycarbonsäure.

**11.** Verwendung gemäss Anspruch 10, dadurch gekennzeichnet, daß die Hydroxysäure ausgewählt ist unter Salicylsäure oder einem ihrer Derivate oder Glykolsäure.

**12.** Verwendung gemäss Anspruch 11, dadurch gekennzeichnet, daß das Salicylsäurederivat 5-n-Octanoylsalicylsäure ist.

**13.** Verwendung gemäss einem der Ansprüche 9 bis 12, dadurch gekennzeichnet, daß das keratolytische Mittel in einer Konzentration zwischen 0,5 und 10 Gew.-% vorhanden ist, bezogen auf das Gesamtgewicht der Zubereitung.

**14.** Verwendung gemäss einem der vorstehenden Ansprüche, dadurch gekennzeichnet, daß die Zubereitung eine Assoziation von Kaffeesäure, Kojisäure und 5-n-Octanoylsalicylsäure enthält.

**15.** Pharmazeutische oder kosmetische Zubereitung mit auf topischem Wege depigmentierender Wirkung, dadurch gekennzeichnet, daß sie in einem geeigneten Träger eine wirksame Menge eines Alkylamids der Kaffeesäure enthält, entsprechend der folgenden allgemeinen Formel:

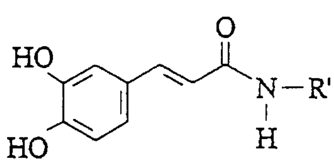
(II)

worin:
R' einen Alkylrest mit 1 bis 8 Kohlenstoffatomen darstellt.

**16.** Zubereitung gemäss Anspruch 15, dadurch gekennzeichnet, daß sie 0,1 bis 8 Gew.-% des Alkylamids der Kaffeesäure enthält, bezogen auf das Gesamtgewicht der Zubereitung.

**17.** Zubereitung gemäss Anspruch 15 oder 16, dadurch gekennzeichnet, daß sie zusätzlich eine Substanz enthält, ausgewählt unter Hydrochinon oder einem Hydrochinonether, Kojisäure, einem wasserlöslichen Kupfersalz oder einer Mischung dieser Substanzen.

**18.** Zubereitung gemäss einem der Ansprüche 15 bis 17, dadurch gekennzeichnet, daß sie Hydrochinon oder einen Hydrochinonether in einer Konzentration zwischen 0,2 und 6 Gew.-% enthält, bezogen auf das Gesamtgewicht der Zubereitung.

**19.** Zubereitung gemäss einem der Ansprüche 15 bis 17, dadurch gekennzeichnet, daß sie die Kojisäure in einer Konzentration zwischen 0,1 und 3 Gew.-% enthält, bezogen auf das Gesamtgewicht der Zubereitung.

**20.** Zubereitung gemäss einem der Ansprüche 15 bis 17, dadurch gekennzeichnet, daß sie das wasserlösliche Kupfersalz in einer moläquivalenten Konzentration zu derjenigen des Alkylamids der Kaffeesäure enthält.

**21.** Zubereitung gemäss einem der Ansprüche 15 bis 20, dadurch gekennzeichnet, daß sie zusätzlich mindestens ein keratolytisches Mittel enthält.

**22.** Zubereitung gemäss Anspruch 21, dadurch gekennzeichnet, daß das keratolytische Mittel ausgewählt ist unter Harnstoff und einer Hydroxycarbonsäure.

**23.** Zubereitung gemäss Anspruch 21 oder 22, dadurch gekennzeichnet, daß das keratolytische Mittel in einer Konzentration zwischen 0,5 und 10 Gew.-% enthalten ist, bezogen auf das Gesamtgewicht der Zubereitung.

**24.** Pharmazeutische oder kosmetische Zubereitung mit einer depigmentierenden Wirkung auf topischem Wege, dadurch gekennzeichnet, daß sie in einem geeigneten Träger eine ausreichende Menge an Kaffeesäure oder einen ihrer Alkylester oder Alkylamide in Verbindung mit einer Substanz enthält, ausgewählt unter Hydrochinon oder einem Hydrochinonether, Kojisäure, einem wasserlöslichen Kupfersalz oder einer Mischung dieser Substanzen.

**25.** Zubereitung gemäss Anspruch 24, dadurch gekennzeichnet, daß sie 0,1 bis 8 Gew.-% Kaffeesäure oder einen ihrer Alkylester oder Alkylamide enthält.

**26.** Zubereitung gemäss Anspruch 24 oder 25, dadurch gekennzeichnet, daß sie 0,2 bis 8 Gew.-% einer Substanz enthält, ausgewählt unter Hydrochinon oder einem Hydrochinonether, Kojisäure, einem wasserlöslichen Kupfersalz oder einer Mischung dieser Substanzen.

**27.** Verfahren zur kosmetischen Behandlung zum Verbessern des ästhetischen Anblicks der Haut, dadurch gekennzeichnet, daß es aus der Anwendung einer ausreichenden Menge einer Zubereitung definiert gemäss einem der Ansprüche 15 bis 26 auf der Haut besteht.

FIG.1

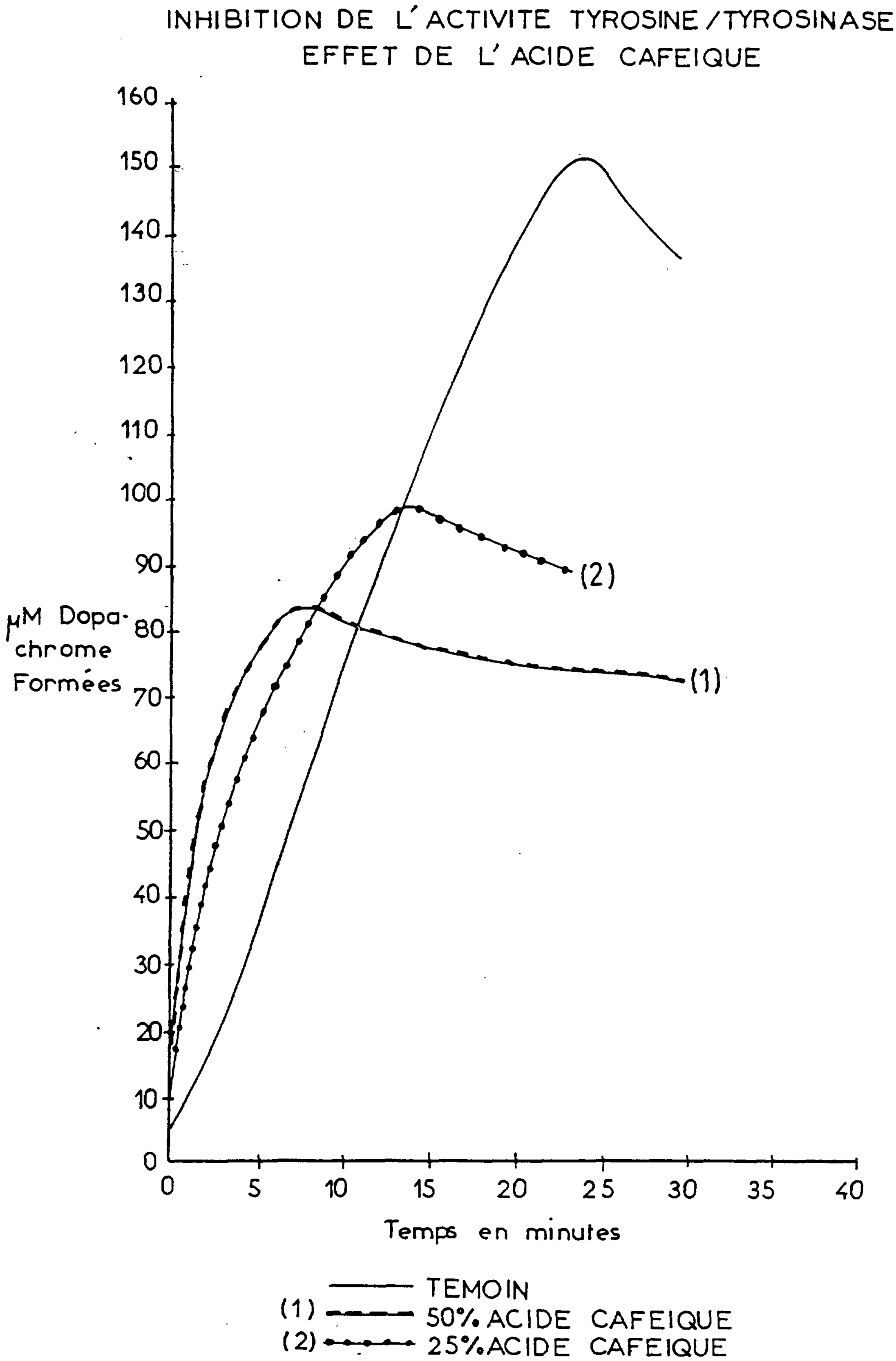
INHIBITION DE L'ACTIVITE TYROSINE/TYROSINASE
EFFET DE L'ACIDE CAFEIQUE
μM Dopa-chrome Formées
160
150
140
130
120
110
100
90
80
70
60
50
40
30
20
10
0
0
5
10
15
20
25
30
35
40
Temps en minutes
(2)
(1)
TEMOIN
(1) 50% ACIDE CAFEIQUE
(2) 25% ACIDE CAFEIQUE

FIG.2

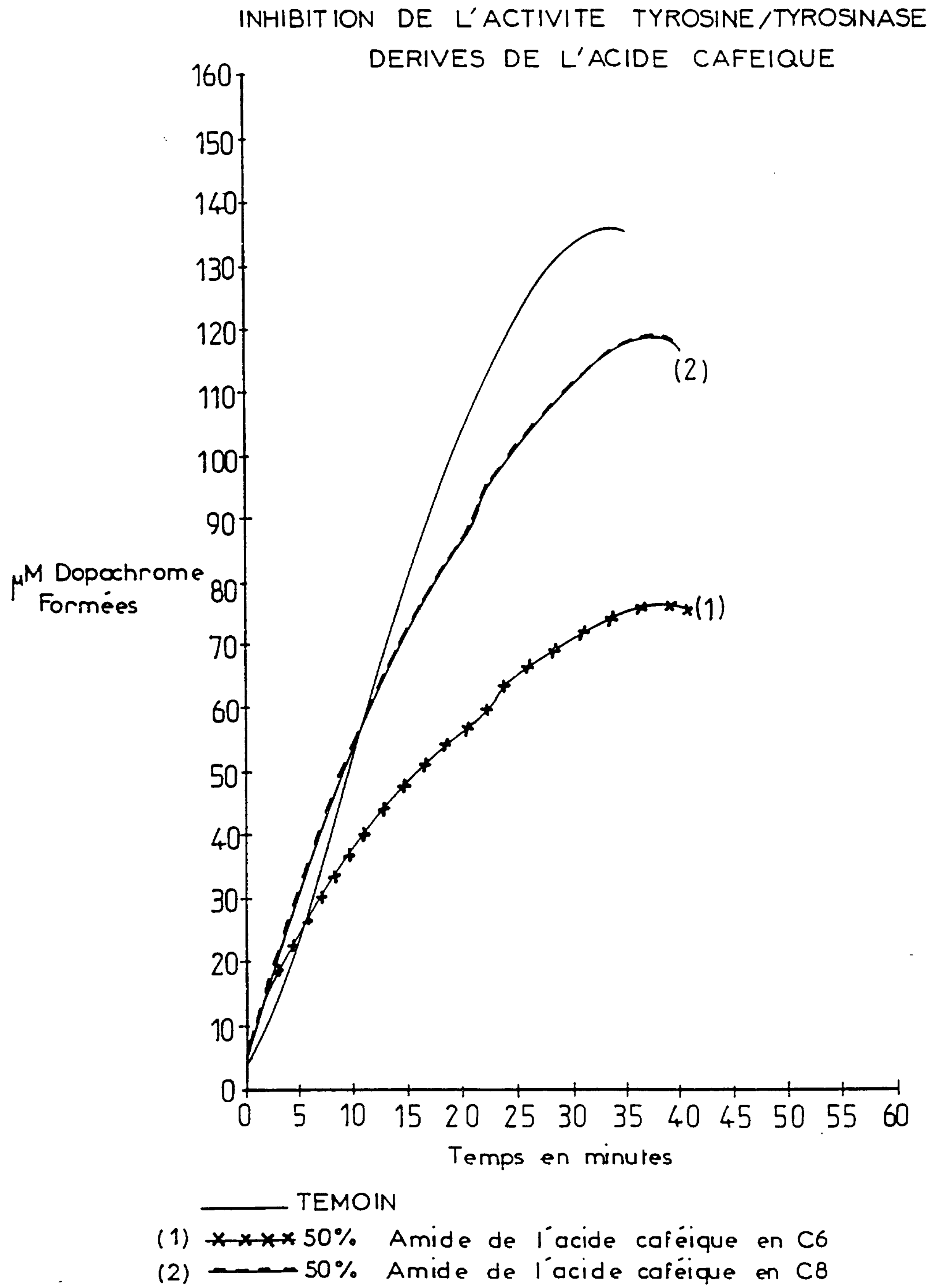
INHIBITION DE L'ACTIVITE TYROSINE/TYROSINASE
DERIVES DE L'ACIDE CAFEIQUE
160
150
140
130
120
110
100
90
80
70
60
50
40
30
20
10
0
μM Dopachrome Formées
(2)
(1)
0 5 10 15 20 25 30 35 40 45 50 55 60
Temps en minutes
TEMOIN
(1) 50% Amide de l'acide caféique en C6
(2) 50% Amide de l'acide caféique en C8

FIG.3

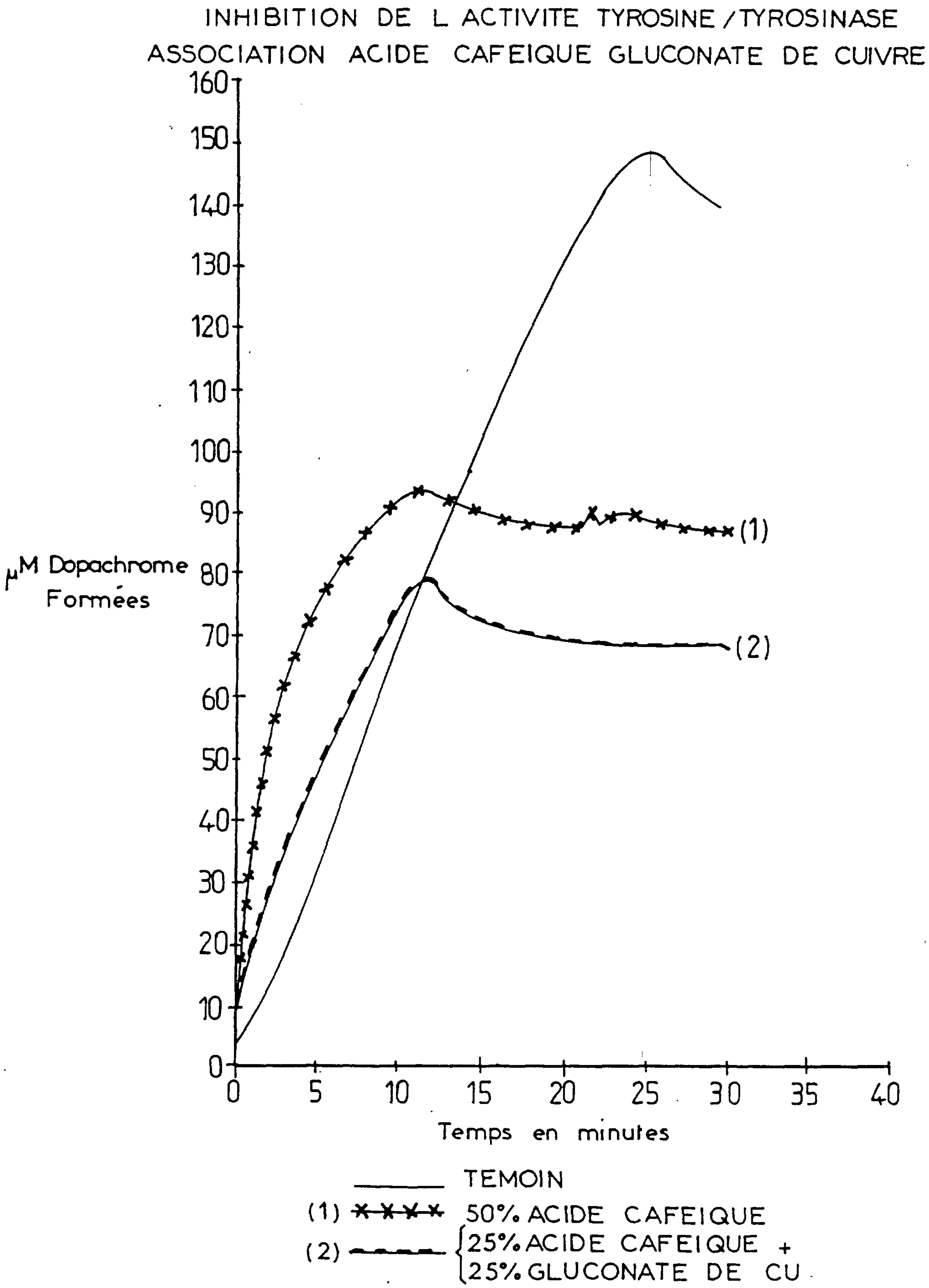
INHIBITION DE L ACTIVITE TYROSINE / TYROSINASE
ASSOCIATION ACIDE CAFEIQUE GLUCONATE DE CUIVRE
μM Dopachrome Formées
160
150
140
130
120
110
100
90
80
70
60
50
40
30
20
10
0
0
5
10
15
20
25
30
35
40
Temps en minutes
(1)
(2)
TEMOIN
(1) 50% ACIDE CAFEIQUE
(2) 25% ACIDE CAFEIQUE + 25% GLUCONATE DE CU

FIG.4

INHIBITION DE L'ACTIVITE TYROSINE / TYROSINASE
ASSOCIATIONS A BASE D'ACIDE CAFEIQUE

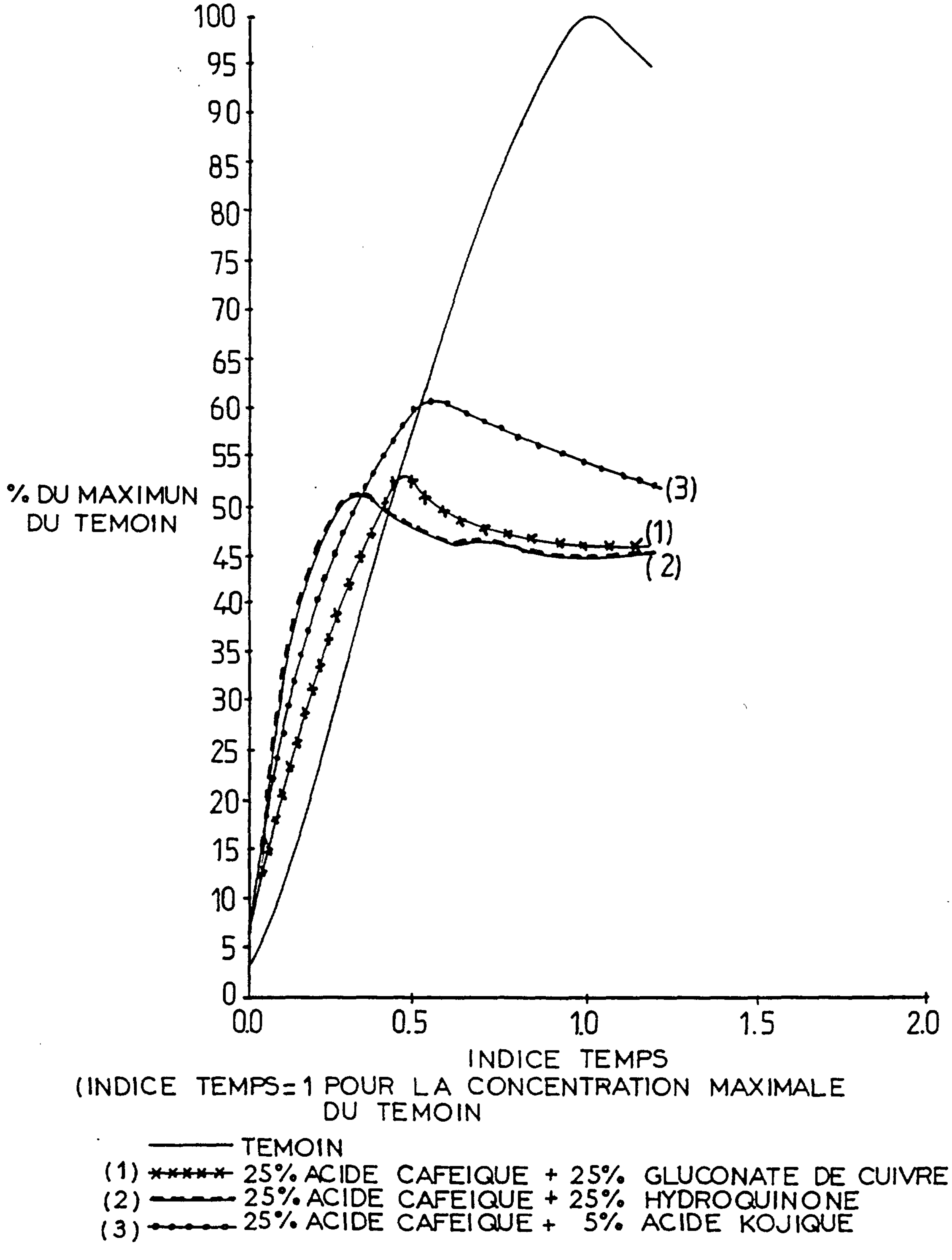

FIG.5

INHIBITION DE L'ACTIVITE TYROSINE/TYROSINASE ASSOCIATIONS A 3 ET 4 COMPOSANTS CONTENANT L'ACIDE CAFEIQUE